# EUROPEAN PATENT SPECIFICATION

(11) **EP 4 164 560 B1**
(45) Date of publication and mention of the grant of the patent: **29.10.2025**
(21) Application number: 21762990.6
(22) Date of filing: 09.09.2021
(51) Int. Cl.: A61F 9/00, A61F 9/007, A61F 2/95

(54) **DELIVERY SHAFT ASSEMBLY FOR DELIVERY OF AN IMPLANT**
EINFÜHRUNGSSCHAFTANORDNUNG ZUR EINFÜHRUNG EINES IMPLANTATS
ENSEMBLE ARBRE DE MISE EN PLACE D'UN IMPLANT

(30) Priority: 15.09.2020 EP 20196252
(43) Date of publication of application: 19.04.2023
(73) Proprietor: iSTAR Medical SA, 1300 Wavre (BE)
(72) Inventor: DETRY, Benoit, 4432 Alleur (BE); UHRLANDT, Stefan, 22587 Hamburg (DE); SEDANO SANTOS, Elena, 1060 Bruxelles (BE); CLEMENCE, Adrien, 1300 Wavre (BE)
(74) Representative: De Clercq & Partners
(86) International application number: PCT/EP2021/074774
(87) International publication number: WO 2022/058232

(56) References cited:
- WO-A1-2017/108498
- US-A1- 2004 089 306
- US-A1- 2007 073 377
- US-A1- 2015 088 065
- US-B1- 10 695 218
- US-B2- 8 758 290
- US-B2- 9 066 828
- US-B2- 9 808 308

## Description

### Field of the invention

Provided herein is a delivery shaft assembly for delivery of an occur implant, for implantation in a subject.

### Background to the invention

Implants are often used to treat conditions where medicinal treatment has become ineffective or is not available. Techniques for placement of an implant typically rely on an inserter tool that temporarily holds the implants and provides an elongated reach for placement at the implantation site and a mechanism for ejection from the inserter.

Implants can be very small, for instance, implants for the treatment of glaucoma typically have a length of 5 mm and a width of 1 mm. An example of an implant and inserter tool for treatment of glaucoma is disclosed, for instance, in WO 2017/108498. This document discloses a delivery shaft assembly having a proximal end and a distal end for delivery of an ocular implant. The shaft assembly comprises a delivery shaft having a lumen configured for holding the implant, and an adapter at the proximal end of the delivery shaft configured for attachment to an inserter tool for deployment of the implant;the delivery shaft comprises a distal compliant section that is repeatably bendable, compliant, and biased in a curve in a 1st plane; the delivery shaft comprises a distal tip section having an atraumatic pointed end; and the delivery shaft is formed from a compliant material formed into a bend-resistive tube.

A problem in the art is how to introduce and deploy the implant with an introducer that is minimally traumatic, yet offers sufficient pushing force and flexibility to enter into the curved tissue of the eye. A further problem is visibility of the implant during surgery; the implant being concealed within an introducer requires a solution for visibility under light microscopy before and during deployment.

### Summary

Provided herein is a delivery shaft assembly (200) having a proximal end (20) and a distal end (40) for delivery of an ocular implant (230), wherein:
- delivery shaft assembly (200) comprises a delivery shaft (220) having a lumen (222) configured for holding the implant (230), and an adapter (210) at the proximal end (20) of the delivery shaft (220) configured for attachment to an inserter tool (500) for deployment of the implant (230);
- the delivery shaft (220) comprises a distal compliant section (244) that is repeatably bendable, compliant, and biased in a curve in a 1^{st} plane (60);
- the delivery shaft (220) is provided with an axially longitudinal observation fenestration (258) allowing visualisation of the implant (230) from an anterior (52) side of the delivery shaft (220);
- the delivery shaft (220) comprises a distal tip section (246) having an atraumatic pointed end;
- the delivery shaft (220) is formed from a compliant material formed into a bend-resistive tube (250), and a wall (224) of the tube (250) in the distal compliant section (244) is provided with a plurality of flexibility slots (252) and/or flexibility apertures (256-b) that impart the bendability.

The compliant material may be an opaque metal, preferably nitinol.

The plurality of flexibility slots (252) and/or flexibility apertures (256-b) may be provided by removal of material from the tube (250) wall (224), preferably by laser cutting.

The delivery shaft (220) may further comprise a proximal section (242) adjacent to the compliant distal section (244), wherein:
- the tube (250) in the proximal section (242) comprises one or more compliant portions (248), or
- a total axial length of the proximal portion (242) is non-compliant and straight.

Each compliant portion (248) may comprise one or more flexibility slots (252)

The observation fenestration (258) may be an axially-longitudinal aperture (256-a) disposed on an anterior (52) side of the tube (250).

The flexibility slots (252) in the distal compliant section (244) may be arranged in a row (262) confined within a posterior (54) half of the transverse cross-section of the tube (250);
- the wall (224) of the tube (250) in the distal compliant section (244) may be further provided with a plurality of flexibility apertures (256-b) known as stent apertures (257, 256-b) that further impart a partial stent-like appearance to the tube (250) and the bendability; and
- the plurality stent apertures (257, 256-b) may be disposed in two axial rows (260', 260"), each row positioned either side of the observation fenestration (258) and between the row (262) of flexibility slots (252).

A majority of or all the stent apertures (257, 256-b) have the same shape, and/or
- a majority of or all the stent apertures (257, 256-b) have the same size, and/or
- a majority of or all the stent apertures (257, 256-b) have a triangle, lozenge, pentagon, hexagon or polygon shape.

The tube (250) in the distal tip section (246) may comprise a pair of axially-longitudinal open slots (270-a, 270-b) extending to the distal terminal end (251) of the tube (250) that define a pair of restricting jaws (272, 274);
- one jaw is an anterior (52) positioned jaw (272) and the other jaw is a posterior (54) positioned jaw (274);
- one or both of the anterior jaw (272) and posterior jaw (274) are angled closed towards a central axis (a-a') of the tube (250); and
- one or both of the anterior jaw (272) and posterior jaw (274) is disposed with a plurality of radial slits that form a living hinge (276).

A closed end of an axially-longitudinal open slot (270-a, 270-b) is in the distal tip section (246).

The tube (250) may comprise a pair of axially-longitudinal open slots (280-a, 280-b) disposed on an anterior (52) and posterior (54) side of the tube (250) and extending to the distal terminal end (251) of the tube (250) that define a pair of retaining arms (282, 284) configured to retain the implant (230) prior to deployment; and
- the observation fenestration (258) is one of the axially-longitudinal open slots (280-a) disposed on an anterior (52) side of the tube (250).

A closed end (253) of an axially-longitudinal open slot (280-a, 280-b) may be in the proximal section (242) or in the distal compliant section (244).

The tube (250) at the distal tip section (246) may be bevelled.

Further provided is a method for manufacturing the delivery shaft assembly (200) as described herein comprising:
- providing the delivery shaft (220) having a proximal (20) end and a distal (20) end comprising the lumen (222) configured for holding the implant (230) wherein the delivery shaft (220) is made from a compliant material formed into a bend-resistive tube (250);
- introducing a curve at the distal end of the delivery shaft (220) thereby forming a distal compliant section (244);
- forming an atraumatic pointed end in the distal tip section (246);
- introducing the plurality of flexibility slots (252) and/or flexibility apertures (256-b) in a distal compliant section (244) to impart the compliance and bendability in the 1^{st} plane (60) by removal of tube material from the tube wall (224);
- introducing the observation fenestration (258) by removal of tube material from the tube wall (224).

The axially-longitudinal open slots (270-a, 270-b, 280-a, 280-b), and/or living hinge (276) may be introduced by a step of removal of tube material from the tube wall (224).

### Figure Legends

FIG. 1 view of a delivery shaft assembly as described herein coupled to an inserter tool. In Panel A prior to deployment, the implant is held in the delivery shaft lumen. In Panel B after deployment, the delivery shaft is withdrawn proximally over the discharge shaft and the implant is released.
**FIG. 2** is a side lateral view of a delivery shaft assembly as described herein
**FIG. 2A** is a transverse-cross-sectional view of the delivery shaft assembly of FIG. 2 through plane A-A'.
**FIG. 2B** is a transverse-cross-sectional view of the delivery shaft assembly of FIG. 2 through plane B-B'.
**FIG. 2C** is a transverse-cross-sectional view of the delivery shaft assembly of FIG. 2 with the perimeter and perimeter length (*pl*) indicated.
**FIG. 2D** is a transverse-cross-sectional view of the delivery shaft assembly of FIG. through a flexibility slot, with the slot length (*sl*) indicated.
**FIG. 3** is a plan view of an implant described herein.
**FIG. 3A** is a transverse cross-section of the implant of FIG. 3 through plane C-C'.
**FIG. 4A** shows detail of a flexibility slot on a tube wall.
**FIG. 4B** shows detail of a flexibility slot with spacer on a tube wall.
**FIG. 4C** shows detail of a flexibility aperture with spacer on a tube wall.
**FIG. 4D** shows detail of an observation fenestration a tube wall.
**FIG. 5** is a side lateral view of an exemplary delivery shaft assembly as described herein, provided with an observation fenestration and flexibility slots in both the distal compliant section.
**FIG. 5A** is a plan view of a part of the delivery shaft assembly of FIG. 5.
**FIG. 5B** shows the delivery shaft assembly of FIG. 5 further provided with flexibility slots in the proximal section.
**FIG. 6** is a side lateral view of an exemplary delivery shaft assembly as described herein, provided with an observation fenestration and flexibility slots in both the proximal and distal compliant section, and flexibility apertures (stent apertures) in the distal compliant section.
**FIG. 6A** is a plan view of a part of the delivery shaft assembly of FIG. 6.
**FIG. 6B** shows the delivery shaft assembly of FIG. 6 further provided with flexibility slots in the proximal section.
**FIG. 6C** is a transverse cross-sectional view of the delivery shaft assembly of FIG. 6, with rows marked for flexibility slots, flexibility apertures (stent apertures), and the observation fenestration.
**FIG. 7** is a side lateral view of an exemplary delivery shaft assembly as described herein, provided with an observation fenestration and flexibility slots in both the proximal and distal compliant section, and with retaining arms.
**FIG. 7A** is a plan view of a part of the delivery shaft assembly of FIG. 7.
**FIG. 7B** is a plan view of a part of the delivery shaft assembly of FIG. 7, wherein an axially-longitudinal open slot is provided with two transverse struts and an axial struts.
**FIG. 7C** shows the delivery shaft assembly of FIG. 7 further provided with flexibility slots in the proximal section.
**FIG. 8** is a side lateral view of an exemplary delivery shaft assembly as described herein, provided with an observation fenestration and flexibility slots in both the proximal and distal compliant section, and with a pair of restricting jaws.
**FIG. 8A** is a plan view of a part of the delivery shaft assembly of FIG. 8.
**FIG. 8B** shows the delivery shaft assembly of FIG. 8 further provided with flexibility slots in the proximal section.

### Detailed description of invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.*, any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The terms "distal" or "distal to" and "proximal" or "proximal to" are used throughout the specification, and are terms generally understood in the field to mean towards (proximal) or away (distal) from the user's (practitioner's) side of an apparatus. Thus, "proximal" or "proximal to" means towards the user's side and, therefore, away from the subject's (patient's) side. Conversely, "distal" or "distal to" means towards the subject's side and, therefore, away from the user's side. The term "subject" refers to the human or animal receiving the implant. The term "user" refers to the person performing the implantation (*e.g.* surgeon, specialist, practitioner).

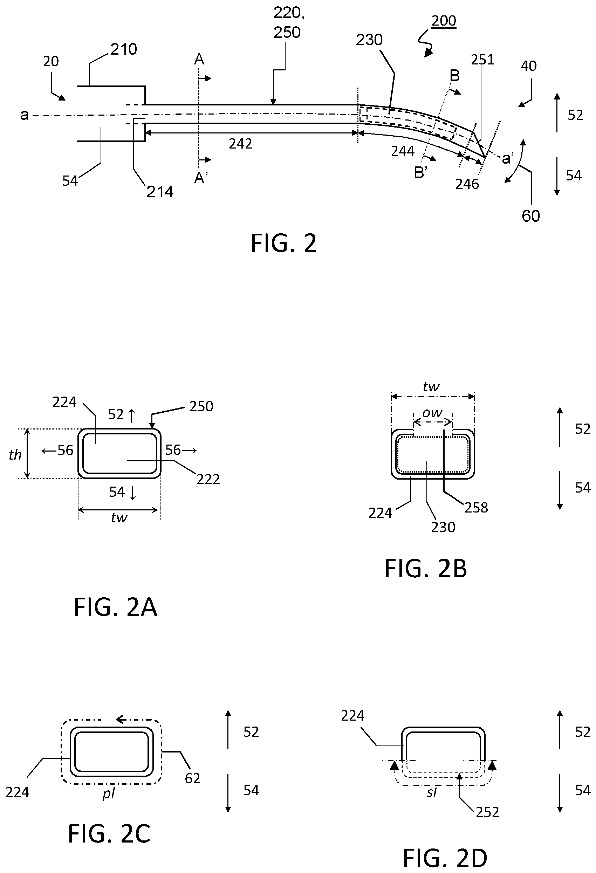

The terms "anterior ", "posterior", and "lateral" are used throughout the specification, and are terms generally understood in the field to mean the front side (anterior) of the subject, the rear-side (posterior) of the subject, and the sides (lateral) of the subject. **FIG. 2A** indicates anterior (52), posterior (54), and lateral (56) sides on a cross-section of the delivery shaft (250). The term "transverse" refers to direction across (*e.g.* perpendicular to) a longitudinal direction. The term "axial" refers to a longitudinal direction of the tube (250), e.g. along axis (a-a'). The term "perimetric" refers to a direction around the exterior of the tube (250), *e.g.* around perimeter (62).

Provided herein is a delivery shaft assembly (200) for delivery of an ocular implant (230) as exemplified for instance in **FIG. 2**, and a method for manufacture of the same. The delivery shaft assembly (200) has a proximal end (20) and a distal end (40) and comprises a delivery shaft (220) having a lumen (222) configured for holding the implant (230).

The delivery shaft (220) comprises a distal compliant section (244) repeatably bendable (60). The distal compliant section (244) is biased in a curve. The curve of the distal compliant section (244) may be in a 1^{st} plane *i.e.* is planar. The bendability of the distal compliant section (244) allows the curved distal compliant section (244) to uncurve and open to a linear form. The compliance of the distal compliant section (244) allows the distal compliant section (244) repeatably to open to a linear form and to return to the curved form. The distal compliant section (244) may bend at least in the 1^{st} plane. The opening to the linear form may be achieved by the application of an external force such as the fixed discharge shaft (510).

The delivery shaft (220) is provided with an observation fenestration (258) (**FIG. 2C**) allowing visualisation of the implant (230) within the delivery shaft (220) from the anterior (52) side by reflective light (*e.g.* under microscopy). The delivery shaft (220) comprises a distal tip section (246) having a reduced transverse profile in a distal (40) direction. The distal compliant section (244) is connected to an adaptor (210) *via* a proximal section (242) of the delivery shaft (220). The proximal section (242) is straight, and may or may not contain one or more compliant portions (248). The delivery shaft (220) is formed from a compliant material formed into a bend-resistive tube (250). A wall (224) of the tube (250) in the compliant portion (240) is provided with a plurality of flexibility slots (252) and/or flexibility apertures (256-b) that impart the bendability(60).

A method for manufacture of the delivery shaft assembly (200) comprises providing a bend-resistive tube (250), and forming the delivery shaft (220) by adding a plurality of flexibility slots (252) and/or flexibility apertures (257) that impart the bendability. The curve of the distal compliant section (244) is formed by any number of ways, for instance, by supporting the lumen (*e.g.* with a flexible coil spring) and bending the tube (250) along its axial length using, for instance, a tube bender. The bending may be performed at ambient temperature, or the bendability of the tube may be enhanced temporarily by heat treatment.

An advantage of the delivery shaft assembly (200) is a delivery shaft (220) having a pushability in an axial (proximal to distal) direction that allows the delivery shaft (220) to be pushed by the user without significant buckling, across the eye. The use of a bend-resistive tube (250) imparts a native stiffness for pushability. The curvature of the distal compliant section (244) allows the distal tip section (246) to follow the curvature of the eye, reducing application of forces caused by pushing to the suprachoroidal space, or subconjunctival space or intrascleral space. The bendability of the distal compliant section (244) allows the passage with reduced force of the straight and fixed discharge shaft (510) through the lumen (222) in the distal compliant section (244) so the implant can be deployed. The bendability of the distal compliant section (244) allows the passage of the straight and fixed discharge shaft (510) through the curved lumen (222) in the distal compliant section (244) so the implant can be deployed. The *compliance* of the distal compliant section (244) allows a reversible deformation of the curve in the distal compliant section (244) so that the distal tip section (246) can actively follow the different radius of curvature of the eye. As the radius of curvature changes during advancement, the advancing distal tip section (246) can reset its position to meet the new form of the advancing curvature. Closely following and adjusting to the tissue landscape reduces intraoperative bleeding.

An advantage of providing an observation fenestration (258) is improved visibility of the implant during deployment. A delivery shaft is typically made from a transparent polymeric material that allows the implant to be seen during insertion. However, because the transparent polymeric material needs to have good pushability, the wall thickness is such that, visibility of the implant or markers thereon is impaired. Increasing the intensity of the light source causes increased reflection which further impairs visualisation.

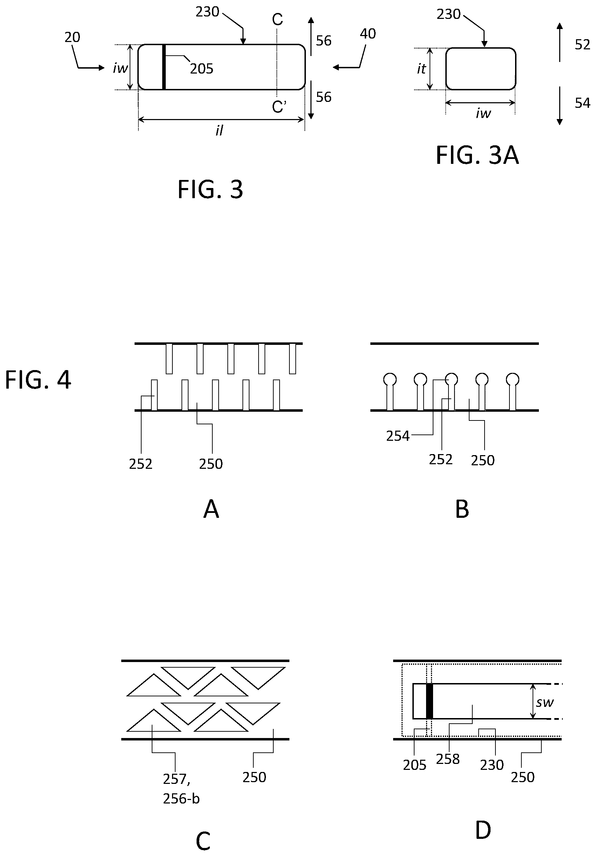

A flexibility slot is a discrete transverse or lateral (56) slot (252) in the wall (224) of the tube (250) that imparts bendability to the tube. A detail of a flexibility slot (252) is shown in **FIG. 4A**. It extends partially around a perimeter (62) of the tube wall (224). A transverse length of the slot (*sl*) is greater than its width (see **FIG. 2D**). The slot path is preferably straight around the perimeter (62) of the tube wall (224). The path of a flexibility slot (252) may coincide with a plane parallel to a central axis (a-a') of the tube (250). One or both ends of a flexibility slot (252) may terminate in a spacer (254) configured to reduce the bending force (see **FIG. 4B**). The spacer (254) may have a circular form.

In a closed flexibility slot (252) the internal edges of the slot form a continuous path. In an open flexibility slot (252) the internal edges of the slot form a discontinuous path; an open end of a flexibility slot (252) may open into for instance, an observation fenestration (258). A majority of or all flexibility slots (252) are spaced apart in an axial direction.

The flexibility slots (252) are preferably arranged in one or more (e.g. preferably two) rows (262) extending in a proximal to distal direction. There may be 4 or more flexibility slots (252) in a row. One or more rows of flexibility slots (252) may be positioned only within a posterior (54) half, or one row flexibility slots (252) may be positioned only within an anterior (52) half of the transverse cross-section. By limiting the flexibility slot to those regions, bending may be limited to the 1^{st} plane, or to a range of planes including or centred on 1^{st} plane.

A flexibility aperture (257) is a discrete closed aperture (256-b) in the wall (224) of the tube (250) that enhances bendability of the tube (250). A flexibility aperture (257) is a closed aperture - the internal edges of the aperture form a continuous path.

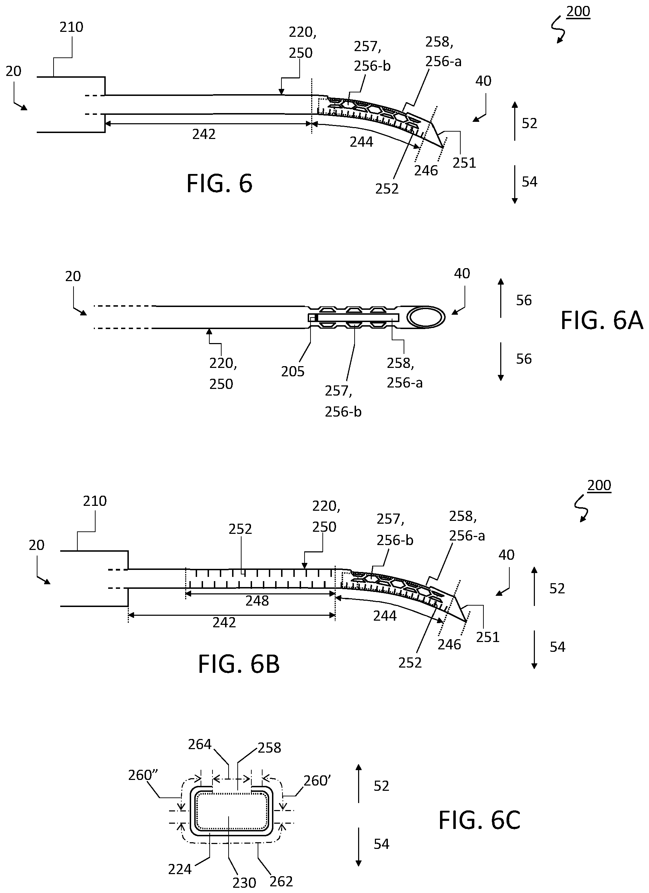

A flexibility aperture may be a stent-aperture (257, 256-b). A stent aperture gives the tube a partially stent-like appearance. A detail of a stent-aperture (257, 256-b) is shown in **FIG. 4C**. Stent apertures (257, 256-b) disposed in a tube wall of a delivery shaft assembly are shown, for instance, in **FIGs. 6** and **6A****.** The stent apertures (257, 256-b) are arranged in one or more (*e.g.* preferably two) rows (262) extending in a proximal to distal direction. The stent apertures (257, 256-b) are spaced apart in an axial direction and in a perimetric direction. A majority of or all the stent apertures (257, 256-b) may have the same shape and/or size. A majority of or all the stent apertures (257, 256-b) may have a triangle, lozenge, pentagon, hexagon or polygon shape. A stent-aperture (257, 256-b) provides lateral visibility of the implant, in addition to enhancing bendability of the tube (250).

The ocular implant (230) (also known as "implant" herein) may be any placeable in a subject (animal, human) using an inserter tool. In particular, the implant (230) may be an implant for treatment of glaucoma. In particular, the implant (230) may be an intraocular shunt. The implant (230) may be for implantation in the treatment target. The treatment target may be between the sclera and the choroid, *i.e.* in the suprachoroidal space, or between the conjunctiva and the sclera (subconjunctival space) or within the sclera (intrascleral space). The implant (230) be an implant as described in WO 2017/108498. In particular, the implant may be as described on page 13, lines 1 to page 14, line 5, and/or on page 15, line 27 to page 16 line 4, and/or on page 21, line 25 to page 22 line 21 of WO 2017/108498.

An exemplary implant (230) is depicted in **FIGs. 3** and **3A**. It has a longitudinal form. It has a proximal (20) and distal (40) end. The long edges may be straight and parallel. The long edges may be rounded. The proximal and distal ends may be planar and parallel. The edges of the ends may be rounded. A transverse cross section (C-C') (**FIG. 3A**) may be longitudinal. The implant (220) may have an oblong form in plan view. The implant (220) may have an oblong form in transverse cross-section view.

The implant may be disposed with one or more markers (205) provided thereon so that a user deploying the shunt or implant can control the depth of the implant in the treatment target with a portion thereof remaining in the anterior chamber. Placement of the shunt or implant within the eye is made using an implantation or deployment device. The shunt or implant (200) may have a marker (205) located in the proximity of its proximal end (20) so that it is possible to see its position through the observation fenestration (258). It will readily be appreciated that other markers (205) may be provided on the implant (200) for better visibility, for example, two markers may be provided at the proximal end (20) of the implant (200).

Typically, the implant (230) have a longitudinal length (*il*) of between 3mm and 9mm. The implant (230) may have a thickness (*it*) of between 0.3mm and 1 mm. The implant (230) may have a width (*iw*) between 0.5mm and 2mm. In a preferred embodiment, the implant is 5mm long, 0.6mm thick and 1.1 mm wide. Dimension indicators are shown in **FIGs. 3** and **3A**.

The implant (200) is located in the distal end (40) of the delivery shaft (240) prior to implantation. Preferably it is located at least partially in the distal compliant section (244). It may be located exclusively in the distal compliant section (244).

The implant may be made from a biocompatible material. The biocompatible material may be that such as that described in EP-B-2517619, which is incorporated hereby reference. It will readily be appreciated that the implant may be made from other suitable biocompatible materials, for example, silicone. In EP-B-2517619, the described biocompatible material is porous and comprises a biocompatible polymer scaffold that defines an array of interconnected pores having similar diameters. Typically, the mean diameter of the pores is between about 20µm and about 90µm, preferably between about 25µm and about 75µm. For use in the implant of the present invention, the preferred range is between about 25µm and about 36µm.

The delivery shaft (220) has a proximal (20) and distal (40) end. The delivery shaft (220) is disposed with an internal longitudinal lumen (222) open at both the proximal (20) and distal (40) ends. The lumen (222) in transverse (A-A', B-B') cross-section has a longitudinal profile (**FIGs. 2A**, **2B**). The lumen (222) in transverse (A-A', B-B') cross-section may have an oblong profile (**FIG. 2A**); the corners may be rounded. Transverse cross-sections of the lumen (222) may have a uniform size and shape along a proximal-distal direction in the compliant portion (240). Transverse cross-sections of the lumen (222) may have a uniform size and shape along a proximal-distal direction in the proximal section (242). Transverse cross-sections of the lumen (222) may have a uniform size and shape along a proximal-distal direction in the distal compliant section (244). The lumen (222) is configured for holding the implant (230).

The proximal (20) end of the delivery shaft (220) is attached to the adaptor (210). As shown in the figures, the proximal end may extend into a body of the adaptor to secure it and for stability.

The delivery shaft (220) is formed from a bend-resistive tube (250). By bend resistive, it is meant exhibiting little or no bending along an axial direction. Factors that contribute to bend-resistivity include wall thickness lumen size, and material of the tube. The skilled person will readily understand how to make a tube bend-resistive, and as a guidance, a tube made from a metal (*e.g.* pure metal, metal alloy, nitinol), having a maximum outer width of 0.96 mm and a wall thickness of 0.05 mm is bend-resistive. The properties of the tube material may include Modulus of Elasticity lower than 310 kpsi. One or more flexibility slots (252) and/or flexibility apertures (257) are added to the tube (250) in order to impart the bendability *e.g.* in the 1^{st} plane (60), or to a range of planes including or centred on the 1^{st} plane.

The use of a metallic bend-resistive tube allows a reduction in the outer diameter of the delivery shaft (220) compared with those formed from polymer. A reduced diameter delivery shaft introduces less damage to the treatment target. Additionally, there is a reduction of the cyclodialysis cleft. Furthermore, a metallic bend-resistive tube can be sterilised *e.g.* by steam and/or immersion in saline under sterilisation conditions (*e.g.* 121 deg C for 30 minutes) and then stored in the saline solution. This reduces manufacturing costs as the delivery shaft (220) can be sterilised without loss of shape or function in the same vessel it will be sterilised and stored in.

The bend-resistive tube (250) is made from a compliant material. By compliant is meant that a leaf spring formed from the material can flexibly deflect from its original shape by application of a mechanical displacing force, and return to its original shape when the mechanical force is released. A material exhibiting a leaf spring property is compliant. When the material is rolled into a tube, the tube is no longer compliant *i.e.* it is bend-resistive. The one or more flexibility slots (252) and/or one or more flexibility apertures (257) added to the tube (250) impart also the bending property to the tube (250), and concomitantly the compliant property. The material might be light opaque. Examples of suitable material include a metals such as nitinol.

As mentioned elsewhere, the delivery shaft (220) comprises a distal compliant section (244). The distal compliant section (244) may have a length between 15 to 35 % of the total exposed delivery shaft (220) length. The distal compliant section (244) is biased in a curve parallel to the 1st plane (60). The delivery shaft (220) further comprises a distal tip section (246).

One or more flexibility slots (252) and/or one or more flexibility apertures (257) and/or observation fenestration (258) (and axially-longitudinal open slots (270-a, 270-b, 280-a, 280-b), and/or living hinge described elsewhere herein) may be introduced into the tube (250) by any method for removing a region of the tube wall (224). Exemplary methods include laser cutting, photochemical etching, deep pressing, conventional chipping techniques such as drilling or milling, high pressure water jet cutting systems or any suitable material removing process available. Preferably laser cutting is used as this allows a very accurate and clean removal of material under reasonable economic conditions.

The delivery shaft (220) and the portions and sections are preferably formed from one piece of tube (250).

The delivery shaft (220) may have a length in an axial (a-a') direction of 35 mm to 40 mm, preferably 34 mm to 37 mm.

The length is measured along a central axis (a-a') from the proximal terminal end of the delivery shaft to the distal terminal end of the distal tip section (246). A few (0.5 to 4 mm) millimetres of the proximal end of the delivery shaft (220) is typically embedded in the distal end of the adaptor (210). The delivery shaft (240) may have a maximum outer tube width across the lateral sides (*tw* in **FIG. 2A**) of 0.9 mm to 1.3 mm, preferably about 1.05 mm to 1.25 mm, more preferably 1.15±0.1 mm. The delivery shaft (240) may have an outer tube height across the posterior-anterior side of (*th* in **FIG. 2A**) of 0.5 mm to 0.7 mm, preferably about 0.52 mm to 1.24 mm.

As mentioned elsewhere herein, the distal compliant section (244) is compliant, repeatably bendable, and biased in a curvee. The curve is in a posterior (54) direction. The curve may in a 1^{st} plane (60). The curve may have a path that is a circular segment. The curve meets a curvature of the eye.

The distal compliant section (244) may have a length along an axial (a-a') direction of 9 to 12 mm. The length is measured along a central axis (a-a') from the distal end of the curve to the proximal end of the distal tip section (246).

The distal compliant section (244) may have a maximum outer tube width across the lateral sides (*tw* in **FIG. 2A**) of 0.9 mm to 1.3 mm, preferably about 1.05 mm to 1.25 mm, more preferably 1.15±0.1 mm. The delivery shaft (240) may have an outer tube height across the posterior-anterior side of (*th* in **FIG. 2A**) of 0.5 mm to 0.8 mm, preferably about 0.52 mm to 0.62mm.

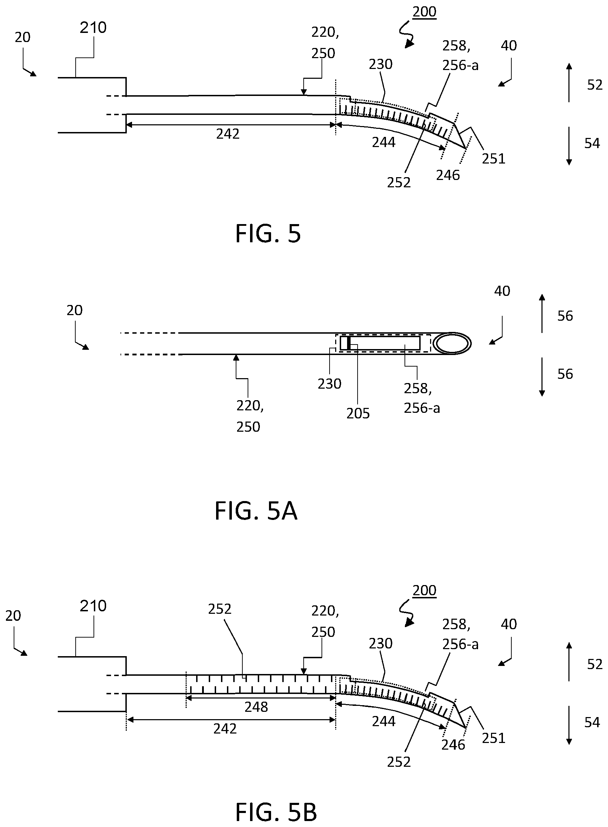

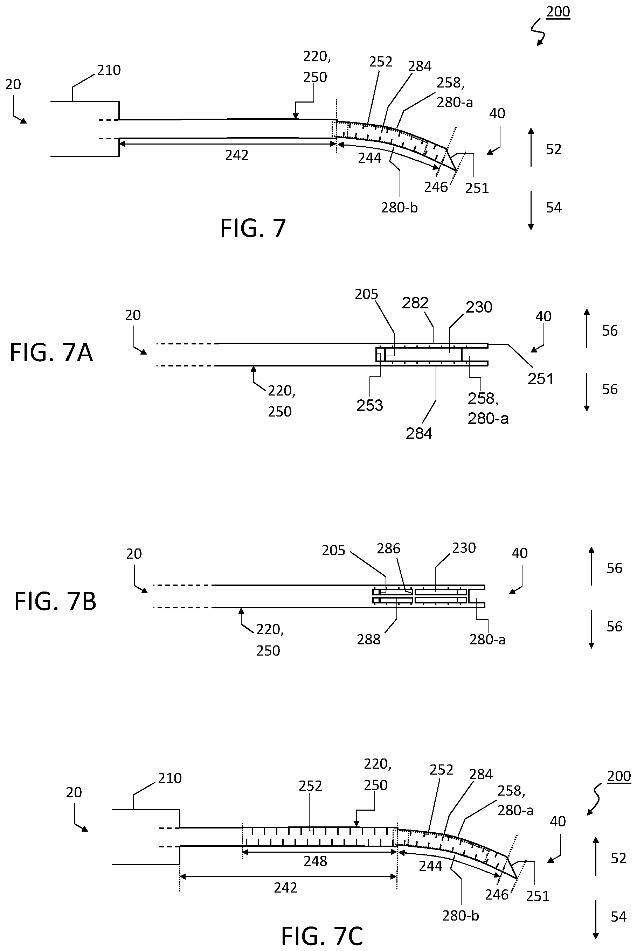

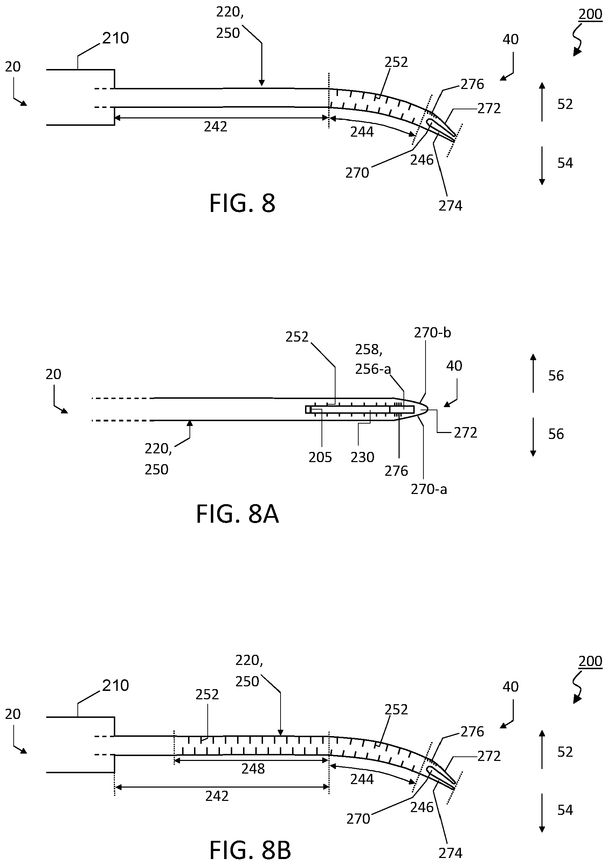

The compliance and bendability of the distal compliant section (244) may be achieved by a plurality flexibility slots (252). A flexibility slot is a discrete transverse or lateral (56) slots (252) in the wall (224) of the tube (250). It extends partially around a perimeter (62) of the tube wall (224). A transverse length of the slot (*sl*) is greater than its width. The slot path is preferably straight. A majority or all of the flexibility slots (252) in the distal compliant section (244) may be closed *i.e.* the internal edges of the slot form a continuous path (*e.g.* **FIGs. 5** and **6**). A majority or all of the flexibility slots (252) in the distal compliant section (244) may be open *i.e.* the internal edges of the slot open out into the observation fenestration (258) (*e.g.* **FIGs. 7** and **8**).

The path of a flexibility slot (252) may coincide with a plane parallel to a central axis (a-a') of the tube (250). One or both ends of a flexibility slot (252) may terminate in a spacer (254) configured to reduced force required to bend the distal compliant section (244) (see **FIG. 4B**). The spacer (254) may have a circular form.

Each flexibility slot (252) in the distal compliant section (244) may span a portion of the transverse cross-section perimeter path (62) of the tube (250) (see **FIG. 2D**). The transverse cross-section perimeter path (also known a perimeter path) (62) of the tube (250) refers to the outer path of the tube along a transverse cross-section (see **FIG. 2C**). The flexibility slot length (*sl*) in the distal compliant section (244) may be a portion of the length (*pl*) of the perimeter path (62) of the tube (250), and may range from 1 to 80 %. The flexibility slot length (*sl*) may be smaller in an axial region containing the observation fenestration (258) compared with without.

A flexibility slot (252) in the distal compliant section (244) may be positioned only within a posterior (54) half or within an anterior half of the transverse cross-section. Each and every flexibility slot (252) in the distal compliant section (244) is spaced apart in an axial direction. The flexibility slots are preferably arranged in one or more (e.g. preferably two) rows (262) extending in a proximal to distal direction.

A majority of or all the flexibility slots (252) in the distal compliant section (244) may be configured to impart the bendability of the distal compliant section (244). The flexibility slots (252) may be arranged such that bendability is at least in the 1^{st} plane, for instance, in a range of planes including or centred on the 1^{st} plane, or is limited to the 1^{st} plane.

The compliance and bendability of the distal compliant section (244) may be further enhanced by a plurality of discrete closed flexibility apertures (256-b) in the wall (224) of the tube (250), known as stent apertures (257) that together give the tube (250) a partially stent-like appearance. A detail of a stent aperture (257, 256-b) is shown in **FIG. 4C**. Stent aperture (257, 256-b) disposed in a tube wall of a delivery shaft assembly are shown in **FIGs. 6** and **6A**.

A majority of or all the stent apertures (257, 256-b) in the distal compliant section (244) is spaced apart in at least in an axial direction. A majority of or all the stent apertures (257, 256-b) are limited to one or more (preferably two) axial rows (260', 260") (see **FIG. 6C**). An axial row (260', 260") is positioned (perimetrically) between the observation fenestration (258) and between a row of flexibility slots (252).

An axial row (260', 260") may span a portion (260', 260") of the transverse cross-section perimeter path (62) of the tube (250) (see **FIG. 6C**). The transverse cross-section perimeter path (also known as perimeter path) (62) of the tube (250) refers to the outer path of the tube along a transverse cross-section (see **FIG. 2C**). Each axial row (260', 260") may be a portion of the length (*pl*) of the perimeter path (62) of the tube (250), for instance, less than 10 %.

A stent aperture (257, 256-b) in the distal compliant section (244) may be positioned only within a lateral (556) section of the transverse cross-section. A majority of or all the stent apertures (257, 256-b) in the distal compliant section (244) is spaced apart in at least in an axial direction.

A majority of or all the stent apertures (257, 256-b) may be regularly spaced apart. A majority of or all the stent apertures (257, 256-b) may have the same shape and/or size. A majority of or all the stent apertures (257, 256-b) may have a triangle, lozenge, pentagon, hexagon or polygon shape.

A majority of or all the stent apertures (257, 256-b) in the distal compliant section (244) may be configured to impart further bendability of the distal compliant section (244).

The presence of stent apertures (257) improve lateral visualisation of the implant during surgery allowing for a safe, "right-first-time" release of the implant.

At least a part of the distal compliant section (244) may be provided with an observation fenestration (258), as described below. The observation fenestration (258) may be limited to and disposed on an anterior (52) side of the tube (250). The observation fenestration (258) may be a closed aperture (256-a) *i.e.* the edges of the aperture form a closed path, or an axially-longitudinal open slot (280-a). The observation fenestration (258) may or may not extend in a proximal direction (20) into the proximal section (242). The observation fenestration (258) may or may not extend in a distal direction (30) into the distal tip section (246).

The distal tip section (246) is an atraumatic pointed end, configured for entry into the treatment target. In particular, it is configured to prise apart tissue, for instance, the sclera and the choroid (suprachoroidal space), or the conjunctiva and the sclera (subconjunctival space) or within the sclera (intrascleral space). The distal tip section (246) is a part of the delivery shaft (220) in which a transverse profile of the tube wall (224) reduces in size, preferably gradually, in a distal (40) direction. The reduction in size may be due to a bevelled tip (*e.g.* **FIGs. 2**, **5**, **5B**, **6**, **6B**, **7**, **7C**), or due to a narrowing of the wall (250) (*e.g.* **FIG. 8B**).

The distal tip section (246) may comprise a bevelled terminal distal end (251) of the tube (250). The bevelled terminal distal end of the tube (250) may be formed from a diagonal cut-off at the distal end of the tube (250). Examples of a distal tip section (246) with a bevelled distal terminal cut are illustrated for instance, in **FIGs. 2**, **5**, **6**, **7**. The bevel is such that an anterior (52) side of the tube (250) lumen (222) is exposed.

The distal tip section (246) may comprise a gradually narrowed terminal distal end of the tube (250). The gradually narrowed terminal distal end of the tube (250) may be formed by introducing a pair of lateral (56) slots open slots (270) at the distal (40) end of the tube (250) thereby forming a pair of jaws (272, 274), and by compressing the jaws (272, 274) together. Examples of a distal tip section (246) with a gradually narrowed terminal distal end of the tube (250) is illustrated for instance, in **FIG. 8**. The distal tip section (246) may be non-compliant or compliant, preferably compliant.

The tube (250) in the distal compliant section (244) may comprise a pair of axially-longitudinal open slots (280-a, 280-b) extending to the distal terminal end (251) of the tube (250) that define a pair of retaining arms (282, 284). The open slot is open at the distal end. The pair of retaining arms (282, 284) is configured to retain the implant prior to deployment. The pair of retaining arms (282, 284) is configured to guide the implant out of the delivery shaft (220) during deployment.

A closed end (253) of an axially-longitudinal open slot (280-a, 280-b) may be in the proximal section (242) or in the distal compliant section (244), preferably in the distal compliant section (244).

The pair of axially-longitudinal open slots (280-a, 280-b) may be opposite each other. The pair of axially-longitudinal open slots (280-a, 280-b) may be diametrically arranged around the tube (250). One axially-longitudinal open slot (280-a) may be disposed on the anterior side (52) of the tube (250) and the other axially-longitudinal open slot (280-b) may be disposed posterior side (54) of the tube (250). The pair of retaining arms (282, 284) may be arranged opposite each other. The pair of retaining arms (282, 284) may be diametrically arranged around the tube (250). Each of the pair of retaining arms (282, 284) may be disposed on each lateral side (56) of the tube (250).

The axially-longitudinal open slot (280-a) disposed on an anterior (52) side of the tube (250) may serve as an observation fenestration (258).

The tube (250) along one or both axially-longitudinal open slots (280-a, 280-b) may each disposed with one or more transverse struts (286) spanning the perimeter of the axially-longitudinal open slots (280-a, 280-b). The tube (250) along one or both axially-longitudinal open slots (280-a, 280-b) may each disposed with one or more axial struts (288) spanning a part of an axial length the axially-longitudinal open slots (280-a, 280-b).

An axial length of one or both axially-longitudinal open slots (280-a, 280-b) may be equal to or greater than an axial length of the implant (230). An axial length of one or both axially-longitudinal open slots (280-a, 280-b) may be at least 4 mm. One or both axially-longitudinal open slots (280-a, 280-b) may or may not extend a length of the tube (250). An upper limit on the axial length may be 8 mm. A perimetric width of each open slots (280-a, 280-b) may be the same or different. A perimetric width of an open slot (280-a, 280-b) may be equal to or less 30- 40% of the total perimeter length *(pl)* of the tube (250). An axially-longitudinal observation fenestration (258) may be formed from the anterior axially-longitudinal open slots (280-a). Examples of a tube (250) provided with a pair of axially-longitudinal open slots (280-a, 280-b) that define a pair of retaining arms (282, 284) is given in **FIGs. 7**, **7A**, **7B**.

As the implant (230) is transparent, visibly during surgery can be problematic. The presence of two opposing axially-longitudinal open slots (280-a, 280-b) allows illumination from below to improve visibility of the whole implant, not just of the marker. Further the distal tip section is formed from less tubing thereby improving the fineness of the tip.

The tube (250) in the distal tip section (246) may comprise a pair of axially-longitudinal open slots (270-a, 270-b) extending to the distal terminal end (251) of the tube (250) that define a pair of restricting jaws (272, 274). The open slots (270-a, 270-b) are open at the distal end. One jaw is an anterior (52) positioned jaw (272) and the other jaw is a posterior (54) positioned jaw (274). A closed end (253) of an axially-longitudinal open slot (270-a, 270-b) may be in the distal compliant section (244) or in the distal tip section (246), preferably in the in the distal tip section (246).

The pair of axially-longitudinal open slots (270-a, 270-b) may be opposite each other. The pair of axially-longitudinal open slots (270-a, 270-b) may be diametrically arranged around the tube (250). Both axially-longitudinal open slots (270-a, 270-b) may be disposed on the lateral sides (56) of the tube (250). The pair of restricting jaws (272, 274) may be arranged opposite each other. The pair of restricting jaws (272, 274) may be diametrically arranged around the tube (250). One restricting jaw (272) may be disposed on the anterior side (52) of the tube (250), the other restricting jaw (274) may be disposed on the posterior side (56) of the tube (250).

One or both of the anterior jaw (272) and posterior jaw (274) may be angled closed towards a central axis (a-a') of the tube (250). One or both of the anterior jaw (272) and posterior jaw (274) may be disposed with a plurality of radial slits that form a living hinge (276). An axial length of one or both axially-longitudinal open slots (270-a, 270-b) may be 1.6 mm to 2 mm. The jaw movement around the hinge is compliant and biased in a closed state. A maximum perimetric width of each of one or both axially-longitudinal open slot (270-a, 270-b) may be equal to or less 30% (*e.g.* 10 % to 20 %) of the total perimeter length (*pl*) of the tube (250). Examples of a tube (250) provided with a pair of axially-longitudinal open slots (270-a, 270-b) that define a pair of restricting jaws (272, 274) is given in **FIGs. 8**, **8A**.

The restricting jaws (272, 274) advantageously provides a blunt-ended distal tip section (246) that mechanically prises apart the treatment tissue upon implant deployment. The pair of surfaces (anterior and posterior) provided by the jaws supports the treatment tissue with a larger surface area compared with a bevelled tip, thereby reducing trauma and bleeding. Furthermore, the closed jaws retain the implant within the lumen, prevent loss of the implant during storage, transport and handling.

As described earlier, at least a part of the delivery shaft (240) may be provided with an axially-longitudinal observation fenestration (258) (see **FIGs. 2B** and **4D**). The observation fenestration (258) comprises a longitudinal opening in the tube wall (224) on the anterior (52) side, which fenestration connects the lumen (222) to the exterior of the tube (250). The observation fenestration (258) allows visualisation of the implant (230), in particular of the marker (205). It allows the implant (230) to be visualised relative to the treatment target prior to deployment. It allows the deployment of implant (230) to visualised. The observation fenestration (258) may be disposed on an anterior (52) side of the tube (250). The observation fenestration (258) may be limited to an anterior (52) side of the tube (250).

The observation fenestration (258) may span a portion of the transverse cross-section perimeter path (62) of the tube (250). The observation fenestration (258) width (ow) may be a portion of the length (*pl*) of the perimeter path (62) of the tube (250), for instance, in a range of 10 to 35 %. In the example of **FIG. 7**, the portion of the observation fenestration (258, 270-a) may be around 30%. In the example of **FIG. 8**, the portion of the observation fenestration (258, 256-a) may be around 14%.

The observation fenestration (258) width (ow) may range from 0.2 mm to 1.0 mm. In the example of **FIG. 5**, the observation fenestration (258) width (ow) may be around 0.6 mm. The observation fenestration (258) width (ow) may range from 0.2 mm to 1.0 mm. In the example of **FIG. 6**, the observation fenestration (258) width (ow) may be around 0.4 mm. In the example of **FIG. 7**, the observation fenestration (258) width (ow) may be around 0.8 mm. In the example of **FIG. 8**, the observation fenestration (258) width (ow) may be around 0.4 mm.

The observation fenestration (258) may be disposed in the distal compliant section (244) and optionally in at least a part of the proximal section (242). The observation fenestration (258) may or may not extend into the distal tip section (246). An axial length of the observation fenestration (258) may be equal to or greater than 6 mm. It may be less than 12 mm. The observation fenestration (258) may be dimensioned to retain the implant (230) prior to and during deployment.

In particular examples, an observation fenestration (258) is an aperture (256-a) as shown for instance, in **FIGs. 5A**, **6A**, **8A**; the edges of the aperture form a closed path. In another example, the observation fenestration (258) is axially-longitudinal open slot (280-a) as shown for instance, in **FIG. 7A**.

The delivery shaft assembly (200) further comprises an adapter (210) configured for coupling to an inserter tool (500). The implant (230) is disposed at or towards a distal end (40) of the delivery shaft (220). The adapter (210) is disposed at a proximal end (20) of the delivery shaft (220). The adapter (210) has a body provided with a receiving space (212) for a part of the inserter tool (500) and an aperture (214) connecting the receiving space (212) to a lumen (222) of delivery shaft (220) (see **FIG. 2**). The lumen (222) is configured for the passage of a discharge shaft (510) of the inserter tool (500) therethrough. The implant (230) may be ejected from the delivery shaft (220) by actuation of the inserter tool (500), thereby delivering the implant (230) to the treatment target. An exemplary delivery shaft assembly (200) is disclosed in WO 2017/108498.

The proximal section (242) is adjacent to the distal compliant section (244) and extends in a proximal direction to the adapter (210).. The proximal section (242) may be straight across its entire axial length. The proximal section (242) of the tube (250) may be bend-resistive across its full axial length. The bend-resistiveness is an inherent property of the bend-resistive tube (250). Examples where the tube (250) in the proximal section is bend-resistive across its full axial length is shown in **FIGs. 5**, **6**, **7**, and **8**.

The proximal section (242) may contain one or more compliant portions (248). The one or more compliant portions (248) may be biased in a straight line. The one or more compliant portions may bend in the is at least in the 1^{st} plane, for instance, in a range of planes including or centred on the 1^{st} plane, or is limited to the 1^{st} plane. Examples where the tube (250) in the proximal section is contains a compliant portion (248) is shown in **FIGs**. **5B**, **6B**, **7C**, and **8B**.

Where one or more compliant portions (248) is present in the proximal section (242), the compliance and bendability is achieved by a plurality of flexibility slots (252). A flexibility slot is a discrete transverse or lateral (56) slot (252) in the wall (224) of the tube (250). It extends partially around a perimeter (62) of the tube wall (224). A transverse length of the slot (*sl*) is greater than its width. The slot path is preferably straight. A majority or all of the flexibility slots (252) in a compliant portion (248) may be closed *i.e*. the internal edges of the slot form a continuous path.

A detail of a flexibility slot (252) is show in **FIG. 4A**. The path of a flexibility slot (252) may coincide with a plane parallel to a central axis (a-a') of the tube (250). One or both ends of a flexibility slot (252) may terminate in a spacer (254) configured to reduced force required to bend the compliant portion (248) (see **FIG. 4B**). The spacer (254) may have a circular form.

Each flexibility slot (252) may span a portion of the transverse cross-section perimeter path (62) of the tube (250) (see **FIG. 2D**). The transverse cross-section perimeter path (also known a perimeter path) (62) of the tube (250) refers to the outer path of the tube along a transverse cross-section (see **FIG. 2C**). The flexibility slot length (*sl*) in the in the compliant portion (248) of the proximal section (242) may be a portion of the length (*pl*) of the perimeter path (62) of the tube (250), for instance, 1 % to 80 %, for instance 1 % to 40%.

A flexibility slot (252) in the compliant portion (248) may be positioned only within a posterior (54) half or only within an anterior (52) half of the transverse cross-section. A majority of or all flexibility slot (252) in the compliant portion (248) is spaced apart in an axial direction. The flexibility slots are preferably arranged in one or more (preferably two) rows extending in a proximal to distal direction.

A majority of or all the flexibility slots (252) in the compliant portion (248) may be arranged such that bendability is at least in the 1^{st} plane, for instance, in a range of planes including or centred on the 1^{st} plane, or is limited to the 1^{st} plane.

At least a part of the proximal section (242) may be provided with an observation fenestration (258), as described above. The observation fenestration (258) may be limited to and disposed an anterior (52) side of the tube (250). The observation fenestration (258) may be a closed aperture (256-a) *i.e.* the edges of the aperture form a closed path, or an axially-longitudinal open slot (280-a). The observation fenestration (258) may extend distally (40) into the distal compliant section (244).

The proximal section (242) may have a length in an axial (a-a') direction of 26 to 30 mm. The length is measured along a central axis (a-a') from the distal end of the adaptor (210) to the proximal end of the distal compliant section (244). The proximal section (242) may have a maximum outer tube width across the lateral sides (*tw* in **FIG. 2A**) of 0.9 mm to 1.5 mm, preferably about 1.05 mm to 1.25 mm, more preferably 1.15±0.1 mm. The delivery shaft (240) may have an outer tube height across the posterior-anterior side of (*th* in **FIG. 2A**) of 0.5 mm to 0.7 mm, preferably about 0.52 mm to 1.24 mm.

At least the delivery shaft (220) and optionally the adaptor (210) may be disposed with a sheath to protect at least the delivery shaft (220) and optionally the adaptor (210). The sheath may be made from a heat-shrink polymer such as polyester heat shrink film.

Provided herein is a method for manufacturing the delivery shaft assembly as described herein. The delivery shaft (220) and the portions and sections are preferably formed from one piece of tube (250). The flexibility slots (252), and/or flexibility apertures (256-b), observation fenestration (258), axially-longitudinal open slots (270-a, 270-b, 280-a, 280-b), and/or living hinge (276), are introduced into the tube (250) any method any method for removal of tube material from the tube wall (224). Exemplary methods include laser cutting, photochemical etching, deep pressing, conventional chipping techniques such as drilling or milling, high pressure water jet cutting systems or any suitable material removing process available. Preferably laser cutting is used as this allows a very accurate and clean removal of material under reasonable economic conditions.

A method for manufacturing the delivery shaft assembly as described herein comprises:
- providing the delivery shaft (220) having a proximal (20) end and a distal (20) end and the lumen (222) configured for holding the implant (230) wherein the delivery shaft (220) is formed from a compliant material formed into a bend-resistive tube (250);
- introducing a curve at the distal end of the delivery shaft (220) thereby forming a distal compliant section (244);
- forming an atraumatic pointed end in the distal tip section (246)
- introducing the plurality of flexibility slots (252) and/or flexibility apertures (256-b) in the distal compliant section (244) to impart the compliance and bendability by removal of tube material from the tube wall (224);
- introducing the observation fenestration (258) by removal of tube material from the tube wall (224).

Other features where present including the flexibility slots (252) in the proximal section (242), flexibility apertures (257), axially-longitudinal open slots (270-a, 270-b, 280-a, 280-b), and/or living hinge (276) may also be introduced by removal of tube material from the tube wall (224).

Provided herein is a delivery shaft assembly (200) manufactured by one of the methods described herein.

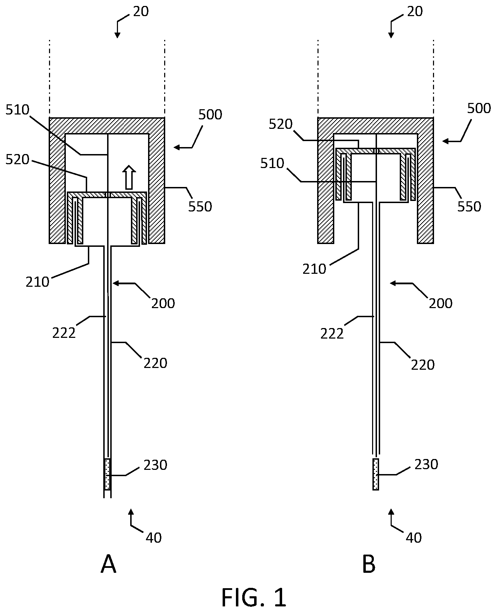

The inserter tool (500) is provided with a discharge shaft (510) configured to be received by the delivery shaft assembly lumen (222). The discharge shaft (510) may be configured to abut the implant (230). Movement of the delivery shaft (220) and/or of the discharge shaft (510) causes release of the implant (230) from the lumen (222). (see **FIG. 1**, **Panels A** and **B**).

Advancement of the discharge shaft (510) in a distal (40) direction may apply force to the implant (230), causing it to be ejected from the shaft assembly lumen (220). Alternatively or additionally, withdrawal of the delivery shaft (220) relative to a fixed discharge shaft (510) causes the implant (230) to be ejected from the shaft assembly lumen (220).

The inserter tool (500) is provided with an adapter coupling (520) configured to couple with the adapter (210). The coupling between the adapter coupling (520) and adapter (210) may be releasable or non-releasable. A non-releasable coupling may be achieved, for instance, by providing a compliant member on one of the adapter coupling (520) or adapter (210) and a reciprocating stop member on the other, wherein the compliant member slides across the stop member in one direction during couple the parts (520, 210) and the compliant member engages with the stop member in the other sliding direction to prevent release of the parts (520, 210). The mechanism is similar to the one-directional movement of a ratchet mechanism.

Where the implant is ejected by withdrawal of the delivery shaft (220) relative to the fixed discharge shaft (510), the adapter coupling (520) may be slidable relative to the fixed discharge shaft (510). The slidable adapter coupling (520) has a primed and deployed position. In the primed position, the slidable adapter coupling (520) places the adapter (210) and delivery shaft lumen (222) in a distal-most position. The fixed discharge shaft (510) may abut the implant (230) and the delivery shaft lumen (222) may cover the implant (230). In the deployed position, the slidable adapter coupling (520) places the adapter (210) and delivery shaft lumen (222) in a proximal-most position. The discharge shaft (510) abuts the implant (230) and the delivery shaft lumen (222) is withdrawn in a proximal (20) direction and the implant (230) is released. Where the implant is ejected by advancement of the discharge shaft (510), the discharge shaft (510) slidable relative to a fixed adapter coupling (520) applied an ejection force in a distal direction upon the implant (230) causing it to be ejected from the lumen (222).

An example of a part of an inserter tool (500) is provided in FIG. 1, showing an inserter housing or chassis (550) (fixed), and a slidable adapter coupling (520) configured to engage with the adapter (210) of the delivery shaft assembly (200). A discharge shaft (510) is disposed in fixed relation to the housing (550). In this example, the adapter coupling (520) is slidable relative to the housing (550) and is shown in a primed position; it is appreciated that the other configurations of the inserter tool (500) exist, for instance, in which the adapter coupling (520) is disposed in fixed relation to an inserter tool housing and the discharge shaft (510) is slidable. In **Panel A**, the inserter tool (500) is shown in the primed position; the adapter coupling (520) is in a distal (40) position. In the primed position, the implant (230) is retained in the delivery shaft lumen (222). In **Panel B**, the inserter tool (500) is shown in the deployed position; the adapter coupling (520) is withdrawn to a proximal (20) position. In the deployed position, the delivery shaft is withdrawn, thereby releasing the implant (230) from the delivery shaft lumen (222). An exemplary inserter tool (500) is disclosed in WO 2017/108498.

The implantation of implant 230 may proceed using, for instance, an *ab interno* method.

The implant (230) is retained within the delivery shaft lumen (222) of the delivery shaft (220) which is mounted on the discharge shaft (510) of the inserter tool (500). The distal tip section (246) may be used to ease penetration of the delivery shaft (220) through the cornea. It will readily be appreciated that the distal tip section (246) does not need to provide the incision into the cornea and this can be done by a separate tool with the delivery shaft (220) being inserted into the incision made. The delivery shaft (220) is directed into and across the anterior chamber of the eye to the iridocorneal angle and into the sub-scleral space. The distal tip section (246 provides an atraumatic penetration into the subscleral space. The delivery shaft (220) is retracted with respect to the discharge shaft (510) of the inserter tool (500) leaving implant (230) in position within the subscleral space.

According to one aspect, a delivery shaft assembly (200) comprises a delivery shaft (220) having a lumen (222) configured for holding the implant (230), wherein:
- the delivery shaft (220) comprises a distal compliant section (244) repeatably bendable, compliant and biased in a curve in a 1^{st} plane (60);
- the delivery shaft (220) comprises a distal tip section (246) having an atraumatic pointed end;
- the delivery shaft (220) is formed from a compliant material formed into a bend-resistive tube (250) and a wall (224) of the tube (250) in the distal compliant section (244) is provided with a plurality of flexibility slots (252) that impart the bendability;
- the flexibility slots (252) in the distal compliant section (244) are arranged in a row (262) confined within a posterior (54) half of the transverse cross-section of the tube (250)
- the tube (250) comprises an observation fenestration (258) that is an axially-longitudinal aperture (256-a) disposed on an anterior (52) side of the tube (250), configured for visualization of at least a part of the implant (230) prior to and during deployment.

An example of the above-mentioned aspect is illustrated in **FIGs. 5** and **5A**. The elements of the above-mentioned aspect have been described elsewhere herein. Certain aspects are highlighted as follows. A majority of preferably all of the flexibility slots (252) are parallel to a plane perpendicular to a central axis (a-a'). The distal tip section (246) may comprise a bevelled terminal distal end (251) of the tube (250).

The axially-longitudinal aperture (256-a) may be disposed at least partially in the distal compliant section (244) and optionally in at least a part of the proximal section (242). The axially-longitudinal aperture (256-a) may not extend into the distal tip section (246).

The flexibility slot length (*sl*) in the distal compliant section (244) may be a portion of the length *(pl)* of the perimeter path (62) of the tube (250), and may range from 25 to 30 % in the axial region of the observation fenestration (258).

The delivery shaft may further comprise a proximal section (242) adjacent to the distal compliant section (244) and extending in a proximal direction to an adapter (210) as described elsewhere. The proximal section (242) may or may not contain one or more compliant portions (248). Each flexibility slot (252) in the compliant portion (248) may have a transverse length (*sl*) that is a portion of a perimeter length (*pl*) of the tube (250), the portion being in a range 1 to 80 %.

A majority of preferably all of the flexibility slots (252) may terminate in a circular spacer (254) configured to reduce the bending force.

According to another aspect, a delivery shaft assembly (200) comprises a delivery shaft (220) having a lumen (222) configured for holding the implant (230), wherein:
- the delivery shaft comprises a distal compliant section (244) repeatably bendable, compliant and biased in a curve in a 1^{st} plane (60);
- the delivery shaft (220) comprises a distal tip section (246) having an atraumatic pointed end;
- the delivery shaft (220) is formed from a compliant material formed into a bend-resistive tube (250) and a wall (224) of the tube (250) in distal compliant section (244) is provided with a plurality of flexibility slots (252) that impart the bendability;
- the tube (250) comprises an observation fenestration (258) that is an axially-longitudinal aperture (256-a) disposed on an anterior (52) side of the tube (250), configured for visualization of at least a part of the implant (230) prior to and during deployment.
- the flexibility slots (252) in the distal compliant section (244) are arranged in a row (262) confined within a posterior (54) half of the transverse cross-section of the tube (250)
- the wall (224) of the tube (250) in the distal compliant section (244) is further provided with a plurality of flexibility apertures (256-b) known as stent apertures (257) that further impart the bendability;
- the plurality of stent apertures (257, 256-b) are disposed in two axial rows (260', 260"), each row positioned either side of the observation fenestration (258) and between the row (262) of flexibility slots (252).

An example of the above-mentioned aspect is illustrated in **FIGs. 6**, **6A** to **6C**. The elements of the above-mentioned aspect have been described elsewhere herein. Certain aspects are highlighted as follows. A majority of preferably all of the flexibility slots (252) are parallel to a plane perpendicular to a central axis (a-a') of the tube (250). The distal tip section (246) may comprise an angled terminal distal end of the tube (250).

The axially-longitudinal aperture (256-a) may be disposed at least partially in the distal compliant section (244) and optionally in at least a part of the proximal section (242). The axially-longitudinal aperture (256-a) may not extend into the distal tip section (246).

The flexibility slot length (*sl*) in the distal compliant section (244) may be a portion of the length (*pl*) of the perimeter path (62) of the tube (250), and may range from 13 to 19 % in the axial region of the stent apertures (257).

The delivery shaft may further comprise a proximal section (242) adjacent to the distal compliant section (244) and extending in a proximal direction to an adapter (210) as described elsewhere. The proximal section (242) may or may not contain one or more compliant portions (248). Each flexibility slot (252) in the compliant portion (248) may have a transverse length (*sl*) that is a portion of a perimeter length (*pl*) of the tube (250), the portion being in a range 1 to 80 %.

A majority of preferably all of the flexibility slots (252) in the proximal section (242) may terminate in a circular spacer (254) configured to reduced force required for bending.

The flexibility apertures (252) may have the same shape *e.g.* polygonal. The presence of stent apertures (257) improve lateral visualisation of the implant during surgery allowing for a safe, "right-first-time" release of the implant.

According to another aspect, a delivery shaft assembly (200) comprises a delivery shaft (220) having a lumen (222) configured for holding the implant (230), wherein:
- the delivery shaft (220) comprises a distal compliant section (244) repeatably bendable, compliant and biased in a curve parallel in a 1^{st} plane (60);
- the delivery shaft (220) comprises a distal tip section (246) having an atraumatic pointed end;
- the delivery shaft (220) is formed from a compliant material formed into a bend-resistive tube (250) and a wall (224) of the tube (250) in the distal compliant section (244) is provided with a plurality of flexibility slots (252) that impart the bendability;
- the tube (250) comprises a pair of axially-longitudinal open slots (280-a, 280-b) disposed on an anterior (52) and posterior (54) side of the tube (250) and extending to the distal terminal end (251) of the tube (250) that define a pair of retaining arms (282, 284) configured to retain the implant (230) prior to deployment;
- the tube (250) comprises an observation fenestration (258) that is the axially-longitudinal open slot (280-a) disposed on an anterior (52) side of the tube (250).

An example of the above-mentioned aspect is illustrated in **FIGs. 7** and **7A**. The elements of the above-mentioned aspect have been described elsewhere herein. Certain aspects are highlighted as follows. A majority or preferably all of the flexibility slots (252) are parallel to a plane perpendicular to a central axis (a-a'). Each flexibility slot (252) in the proximal section (242) may be closed. Each flexibility slot (252) in the distal compliant section (244) may be open. The distal tip section (246) may comprise an angled terminal distal end of the tube (250).

A closed end (253) of an axially-longitudinal open slot (280-a, 280-b) may be in the proximal section (242) or in the distal compliant section (244), preferably in the distal compliant section (244). A maximum perimetric width of each of one or both axially-longitudinal open slot (280-a, 280-b) may be 30 to 40 % of the total perimeter length (pl) of the tube (250).

The flexibility slot length (*sl*) in the distal compliant section (244) may be a portion of the length (*pl*) of the perimeter path (62) of the tube (250), and may range from 8 to 14 % within the axial region of the axially-longitudinal open slot (280-a, 280-b).

The flexibility slot length (*sl*) in the distal compliant section (244) may be a portion of the length (*pl*) of the perimeter path (62) of the tube (250), and may range from 1 % to 80 %, for instance 31 % to 37 %.

The delivery shaft may further comprise a proximal section (242) adjacent to the distal compliant section (244) and extending in a proximal direction to an adapter (210) as described elsewhere. The proximal section (242) may or may not contain one or more compliant portions (248). Each flexibility slot (252) in the compliant portion (248) may have a transverse length (*sl*) that is a portion of a perimeter length (*pl*) of the tube (250), the portion being in a range 1 to 80 %.

A majority of preferably all of the flexibility slots (252) may terminate in a circular spacer (254) configured to reduced force required for bending. As the implant (230) is transparent, visibly during surgery can be problematic. The presence of two opposing axially-longitudinal open slots (280-a, 280-b) allows illumination from below to improve visibility of the whole implant, not just of the marker. Further the distal tip section is formed from less tubing thereby improving the fineness of the tip.

According to another aspect, a delivery shaft assembly (200) comprises a delivery shaft (220) having a lumen (222) configured for holding the implant (230), wherein:
- the delivery shaft (220) comprises a distal compliant section (244) repeatably bendable, compliant and biased in a curve in a 1^{st} plane (60);
- the delivery shaft (220) comprises a distal tip section (246) having an atraumatic pointed end;
- the delivery shaft (220) is formed from a compliant material formed into a bend-resistive tube (250) and a wall (224) of the tube (250) in the proximal section (242) and in distal compliant section (244) is provided with a plurality of flexibility slots (252) that impart the bendability;
- the tube in the comprises an observation fenestration (258) that is an axially-longitudinal aperture (256-a) disposed on an anterior (52) side of the tube (250), configured for visualization of at least a part of the implant (230) prior to and during deployment,
- the tube (250) in the distal tip section (246) comprises a pair of axially-longitudinal open slots (270-a, 270-b) extending to the distal terminal end (251) of the tube (250) that define a pair of restricting jaws (272, 274);
- one jaw is an anterior (52) positioned jaw (272) and the other jaw is a posterior (54) positioned jaw (274);
- one or both of the anterior jaw (272) and posterior jaw (274) are angled closed towards a central axis (a-a') of the tube (250);
- one or both of the anterior jaw (272) and posterior jaw (274) is disposed with a plurality of radial slits that form a living hinge (276).

An example of the above-mentioned aspect is illustrated in **FIGs. 8** and **8A**. The elements of the above-mentioned aspect have been described elsewhere herein. Certain aspects are highlighted as follows. A majority of preferably all of the flexibility slots (252) are parallel to a plane perpendicular to a central axis (a-a') of the tube (250).

A closed end (253) of an axially-longitudinal open slot (270-a, 270-b) may be in the distal tip section (246) or in the proximal section (242), preferably in the distal tip section (246). The jaw movement around the hinge is compliant and biased in a closed state. A maximum perimetric width of each of one or both axially-longitudinal open slot (270-a, 270-b) may be 10 to 20 % of the total perimeter length (pl) of the tube (250).

The flexibility slot length (*sl*) in the distal compliant section (244) may be a portion of the length (*pl*) of the perimeter path (62) of the tube (250), and may range from 1 % to 80 %, for instance 31 % to 37 %.

Each flexibility slot (252) in the distal compliant section (244) is confined within a posterior (54) half or an anterior half (52) of the transverse cross-section of the tube (250). A majority of preferably all of the flexibility slots (252) are parallel to a plane perpendicular to a central axis (a-a').

The axially-longitudinal aperture (256-a) may be disposed at least partially in the distal compliant section (244) and optionally in at least a part of the proximal section (242). The axially-longitudinal aperture (256-a) may not extend into the distal tip section (246).

The delivery shaft may further comprise a proximal section (242) adjacent to the distal compliant section (244) and extending in a proximal direction to an adapter (210) as described elsewhere. The proximal section (242) may or may not contain one or more compliant portions (248). Each flexibility slot (252) in the compliant portion (248) may have a transverse length (*sl*) that is a portion of a perimeter length (*pl*) of the tube (250), the portion being in a range 1 to 80 %.

A majority or preferably all of the flexibility slots (252) may terminate in a circular spacer (254) configured to reduced force required for bending.

The inserter tool (500) may be an implantation device as described in WO 2017/108498.

The delivery shaft assembly (200) may comprise a one-touch fitting connection element or adaptor (210) as described in WO 2017/108498. For example, WO 2017/108498 describes an implantation device (500) and delivery shaft assembly (200) on pages 26 to page 33.

## Claims

1. A delivery shaft assembly (200) having a proximal end (20) and a distal end (40) for delivery of an ocular implant (230), wherein:
- delivery shaft assembly (200) comprises a delivery shaft (220) having a lumen (222) configured for holding the implant (230), and an adapter (210) at the proximal end (20) of the delivery shaft (220) configured for attachment to an inserter tool (500) for deployment of the implant (230);
- the delivery shaft (220) comprises a distal compliant section (244) that is repeatably bendable, compliant, and biased in a curve in a 1^{st} plane (60);
- the delivery shaft (220) is provided with an axially longitudinal observation fenestration (258) allowing visualisation of the implant (230) from an anterior (52) side of the delivery shaft (220);
- the delivery shaft (220) comprises a distal tip section (246) having an atraumatic pointed end; and
- the delivery shaft (220) is formed from a compliant material formed into a bend-resistive tube (250), and a wall (224) of the tube (250) in the distal compliant section (244) is provided with a plurality of flexibility slots (252) and/or flexibility apertures (256-b) that impart the bendability.

2. The delivery shaft assembly (200) according to claim 1, wherein the compliant material is an opaque metal, preferably nitinol.

3. The delivery shaft assembly (200) according to claim 1 or 2, wherein the plurality of flexibility slots (252) and/or flexibility apertures (256-b) are provided by removal of material from the tube (250) wall (224), preferably by laser cutting.

4. The delivery shaft assembly (200) according to any one of claims 1 to 3, wherein the delivery shaft (220) further comprises a proximal section (242) adjacent to the compliant distal section (244), wherein:
- the tube (250) in the proximal section (242) comprises one or more compliant portions (248), or
- a total axial length of the proximal portion (242) is non-compliant and straight.

5. The delivery shaft assembly (200) according to any one of claims 1 to 4, wherein each compliant portion (248) comprises one or more flexibility slots (252)

6. The delivery shaft assembly (200) according to any one of claims 1 to 5, wherein the observation fenestration (258) is an axially-longitudinal aperture (256-a) disposed on an anterior (52) side of the tube (250).

7. The delivery shaft assembly (200) according to any one of claims 1 to 6, wherein:
- the flexibility slots (252) in the distal compliant section (244) are arranged in a row (262) confined within a posterior (54) half of the transverse cross-section of the tube (250)
- the wall (224) of the tube (250) in the distal compliant section (244) is further provided with a plurality of flexibility apertures (256-b) known as stent apertures (257, 256-b) that further impart a partial stent-like appearance to the tube (250) and the bendability;
- the plurality stent apertures (257, 256-b) is disposed in two axial rows (260', 260"), each row positioned either side of the observation fenestration (258) and between the row (262) of flexibility slots (252).

8. The delivery shaft assembly (200) according to claim 7, wherein:
- a majority of or all the stent apertures (257, 256-b) have the same shape, and/or
- a majority of or all the stent apertures (257, 256-b) have the same size, and/or
- a majority of or all the stent apertures (257, 256-b) have a triangle, lozenge, pentagon, hexagon or polygon shape.

9. The delivery shaft assembly (200) according to any one of claims 1 to 8, wherein:
- the tube (250) in the distal tip section (246) comprises a pair of axially-longitudinal open slots (270-a, 270-b) extending to the distal terminal end (251) of the tube (250) that define a pair of restricting jaws (272, 274);
- one jaw is an anterior (52) positioned jaw (272) and the other jaw is a posterior (54) positioned jaw (274);
- one or both of the anterior jaw (272) and posterior jaw (274) are angled closed towards a central axis (a-a') of the tube (250); and
- one or both of the anterior jaw (272) and posterior jaw (274) is disposed with a plurality of radial slits that form a living hinge (276).

10. The delivery shaft assembly (200) according to claim 9, wherein a closed end of an axially-longitudinal open slot (270-a, 270-b) is in the distal tip section (246).

11. The delivery shaft assembly (200) according to any one of claims 1 to 5, wherein:
- the tube (250) comprises a pair of axially-longitudinal open slots (280-a, 280-b) disposed on an anterior (52) and posterior (54) side of the tube (250) and extending to the distal terminal end (251) of the tube (250) that define a pair of retaining arms (282, 284) configured to retain the implant (230) prior to deployment;
- the observation fenestration (258) is one of the axially-longitudinal open slots (280-a) disposed on an anterior (52) side of the tube (250).

12. The delivery shaft assembly (200) according to claim 11 wherein a closed end (253) of an axially-longitudinal open slot (280-a, 280-b) is in the proximal section (242) or in the distal compliant section (244).

13. The delivery shaft assembly (200) according to any one of claims 1 to 12, wherein the tube (250) at the distal tip section (246) is bevelled.

14. A method for manufacturing the delivery shaft assembly (200) according to any one of claims 1 to 13 comprising:
- providing the delivery shaft (220) having a proximal (20) end and a distal (20) end comprising the lumen (222) configured for holding the implant (230) wherein the delivery shaft (220) is made from a compliant material formed into a bend-resistive tube (250);
- introducing a curve at the distal end of the delivery shaft (220) thereby forming a distal compliant section (244);
- forming an atraumatic pointed end in the distal tip section (246);
- introducing the plurality of flexibility slots (252) and/or flexibility apertures (256-b) in a distal compliant section (244) to impart the compliance and bendability in the 1^{st} plane (60) by removal of tube material from the tube wall (224);
- introducing the observation fenestration (258) by removal of tube material from the tube wall (224).

15. The method according to claim 14, wherein the axially-longitudinal open slots (270-a, 270-b, 280-a, 280-b), and/or living hinge (276) are introduced by a step of removal of tube material from the tube wall (224).

## Patentansprüche

1. Zuführschaftbaugruppe (200) mit einem proximalen Ende (20) und einem distalen Ende (40) zum Zuführen eines Augenimplantats (230), wobei:
- die Zuführschaftbaugruppe (200) einen Zuführschaft (220) mit einem Lumen (222), das zum Halten des Implantats (230) ausgestaltet ist, und einen Adapter (210) an dem proximalen Ende (20) des Zuführschafts (220) umfasst, der zum Anbringen an einem Einführwerkzeug (500) zum Einsetzen des Implantats (230) ausgestaltet ist,
- der Zuführschaft (220) einen distalen nachgiebigen Abschnitt (244) umfasst, der wiederholt biegbar, nachgiebig und in einer Krümmung in einer 1. Ebene (60) vorgespannt ist,
- der Zuführschaft (220) mit einem axial längsgerichteten Beobachtungsfenster (258) versehen ist, das eine Visualisierung des Implantats (230) von einer vorderen (52) Seite des Zuführschafts (220) aus gestattet,
- der Zuführschaft (220) einen distalen Spitzenabschnitt (246) mit einem atraumatischen spitzen Ende umfasst und
- der Zuführschaft (220) aus einem nachgiebigen Material gebildet ist, das zu einem biegefesten Rohr (250) geformt ist, und eine Wand (224) des Rohrs (250) in dem distalen nachgiebigen Abschnitt (244) mit einer Vielzahl von Flexibilitätsschlitzen (252) und/oder Flexibilitätsöffnungen (256-b) versehen ist, die die Biegsamkeit verleihen.

2. Zuführschaftbaugruppe (200) nach Anspruch 1, wobei das nachgiebige Material ein opakes Metall, vorzugsweise Nitinol, ist.

3. Zuführschaftbaugruppe (200) nach Anspruch 1 oder 2, wobei die Vielzahl von Flexibilitätsschlitzen (252) und/oder Flexibilitätsöffnungen (256-b) durch Entfernen von Material von der Wand (224) des Rohrs (250), vorzugsweise durch Laserschneiden, bereitgestellt werden.

4. Zuführschaftbaugruppe (200) nach einem der Ansprüche 1 bis 3, wobei der Zuführschaft (220) ferner einen proximalen Abschnitt (242) umfasst, der dem nachgiebigen distalen Abschnitt (244) benachbart ist, wobei:
- das Rohr (250) in dem proximalen Abschnitt (242) einen oder mehrere nachgiebige Abschnitte (248) umfasst oder
- eine axiale Gesamtlänge des proximalen Abschnitts (242) nicht nachgiebig und gerade ist.

5. Zuführschaftbaugruppe (200) nach einem der Ansprüche 1 bis 4, wobei jeder nachgiebige Abschnitt (248) einen oder mehrere Flexibilitätsschlitze (252) umfasst.

6. Zuführschaftbaugruppe (200) nach einem der Ansprüche 1 bis 5, wobei das Beobachtungsfenster (258) eine axial längsgerichtete Öffnung (256-a) ist, die auf einer vorderen (52) Seite des Rohrs (250) angeordnet ist.

7. Zuführschaftbaugruppe (200) nach einem der Ansprüche 1 bis 6, wobei:
- die Flexibilitätsschlitze (252) in dem distalen nachgiebigen Abschnitt (244) in einer Reihe (262) angeordnet sind, die auf eine hintere (54) Hälfte des Querschnitts des Rohrs (250) beschränkt ist,
- die Wand (224) des Rohrs (250) in dem distalen nachgiebigen Abschnitt (244) ferner mit einer Vielzahl von Flexibilitätsöffnungen (256-b) versehen ist, die als Stentöffnungen (257, 256-b) bekannt sind und dem Rohr (250) ferner ein teilstentartiges Aussehen und die Biegsamkeit verleihen,
- die Vielzahl von Stentöffnungen (257, 256-b) in zwei axialen Reihen (260', 260") angeordnet sind, wobei jede Reihe zu beiden Seiten des Beobachtungsfensters (258) und zwischen der Reihe (262) von Flexibilitätsschlitzen (252) positioniert ist.

8. Zuführschaftbaugruppe (200) nach Anspruch 7, wobei:
- ein Großteil der oder alle Stentöffnungen (257, 256-b) die gleiche Form haben und/oder
- ein Großteil der oder alle Stentöffnungen (257, 256-b) dieselbe Größe haben und/oder
- ein Großteil der oder alle Stentöffnungen (257, 256-b) eine Dreieck-, Rauten-, Fünfeck-, Sechseck- oder Polygonform haben.

9. Zuführschaftbaugruppe (200) nach einem der Ansprüche 1 bis 8, wobei:
- das Rohr (250) in dem distalen Spitzenabschnitt (246) ein Paar axial längsgerichteter offener Schlitze (270-a, 270-b) umfasst, die sich zu dem distalen Abschlussende (251) des Rohrs (250) erstrecken und ein Paar Begrenzungsbacken (272, 274) definieren,
- eine Backe eine vordere (52) positionierte Backe (272) und die andere Backe eine hintere (54) positionierte Backe (274) ist,
- die vordere Backe (272) und/oder die hintere Backe (274) zu einer Mittelachse (a-a') des Rohrs (250) hin geschlossen abgewinkelt sind und
- die vordere Backe (272) und/oder die hintere Backe (274) mit einer Vielzahl von radialen Schlitzen angeordnet sind, die ein Filmscharnier (276) bilden.

10. Zuführschaftbaugruppe (200) nach Anspruch 9, wobei sich ein geschlossenes Ende eines axial längsgerichteten offenen Schlitzes (270-a, 270-b) in dem distalen Spitzenabschnitt (246) befindet.

11. Zuführschaftbaugruppe (200) nach einem der Ansprüche 1 bis 5, wobei:
- das Rohr (250) ein Paar axial längsgerichteter offener Schlitze (280-a, 280-b) umfasst, die an einer vorderen (52) und hinteren (54) Seite des Rohrs (250) angeordnet sind und sich zu dem distalen Abschlussende (251) des Rohrs (250) erstrecken und ein Paar Haltearme (282, 284) definieren, die zum Halten des Implantats (230) vor dem Einsetzen ausgestaltet sind,
- das Beobachtungsfenster (258) einer der axial längsgerichteten offenen Schlitze (280-a) ist, die an einer vorderen (52) Seite des Rohrs (250) angeordnet sind.

12. Zuführschaftbaugruppe (200) nach Anspruch 11, wobei sich ein geschlossenes Ende (253) eines axial längsgerichteten offenen Schlitzes (280-a, 280-b) in dem proximalen Abschnitt (242) oder in dem distalen nachgiebigen Abschnitt (244) befindet.

13. Zuführschaftbaugruppe (200) nach einem der Ansprüche 1 bis 12, wobei das Rohr (250) an dem distalen Spitzenabschnitt (246) abgeschrägt ist.

14. Verfahren zur Herstellung der Zuführschaftbaugruppe (200) nach einem der Ansprüche 1 bis 13, umfassend:
- Bereitstellen des Zuführschafts (220) mit einem proximalen (20) Ende und einem distalen (20) Ende, das das Lumen (222) umfasst, das zum Halten des Implantats (230) ausgestaltet ist, wobei der Zuführschaft (220) aus einem nachgiebigen Material hergestellt ist, das zu einem biegefesten Rohr (250) ausgebildet ist,
- Einführen einer Krümmung an dem distalen Ende des Zuführschafts (220), wodurch ein distaler nachgiebiger Abschnitt (244) gebildet wird,
- Bilden einer atraumatischen Spitze im distalen Spitzenabschnitt (246),
- Einführen der Vielhzahl von Flexibilitätsschlitzen (252) und/oder Flexibilitätsöffnungen (256-b) in einen distalen nachgiebigen Abschnitt (244), um die Nachgiebigkeit und Biegsamkeit in der 1. Ebene (60) durch Entfernen von Rohrmaterial von der Rohrwand (224) zu verleihen,
- Einführung des Beobachtungsfensters (258) durch Entfernung von Rohrmaterial von der Rohrwand (224).

15. Verfahren nach Anspruch 14, wobei die axial längsgerichteten offenen Schlitze (270-a, 270-b, 280-a, 280-b) und/oder das Filmscharnier (276) durch einen Schritt des Entfernens von Rohrmaterial von der Rohrwand (224) eingeführt werden.

## Revendications

1. Ensemble formant tige de pose (200) comportant une extrémité proximale (20) et une extrémité distale (40) pour la pose d'un implant oculaire (230), dans lequel :
- l'ensemble tige de pose (200) comprend une tige de pose (220) comportant une lumière (222) conçue pour maintenir l'implant (230), et un adaptateur (210) au niveau de l'extrémité proximale (20) de la tige de pose (220) conçu pour être fixé à un outil d'insertion (500) pour la mise en place de l'implant (230) ;
- la tige de pose (220) comprend une section distale flexible (244) qui est déformable de manière répétée, flexible et sollicitée de façon à définir une courbe dans un 1^{er} plan (60) ;
- la tige de pose (220) est pourvue d'une ouverture d'observation axialement longitudinale (258) permettant de voir l'implant (230) depuis un côté antérieur (52) de la tige de pose (220) ;
- la tige de pose (220) comprend une section de pointe distale (246) comportant une extrémité en pointe atraumatique ; et
- la tige de pose (220) est composée d'un matériau flexible mis sous forme de tube résistant à la déformation (250), et une paroi (224) du tube (250) dans la section distale flexible (244) est pourvue d'une pluralité de fentes de flexibilité (252) et/ou d'orifices de flexibilité (256-b) qui confèrent la déformabilité.

2. Ensemble formant tige de pose (200) selon la revendication 1, dans lequel le matériau flexible est un métal opaque, de préférence le nitinol.

3. Ensemble formant tige de pose (200) selon la revendication 1 ou 2, dans lequel la pluralité de fentes de flexibilité (252) et/ou d'orifices de flexibilité (256-b) sont formés par un enlèvement de matière au niveau de la paroi (224) du tube (250), de préférence par découpe par laser.

4. Ensemble formant tige de pose (200) selon l'une quelconque des revendications 1 à 3, dans lequel la tige de pose (220) comprend, en outre, une section proximale (242) adjacente à la section distale flexible (244), dans lequel :
- le tube (250), dans la section proximale (242), comprend une ou plusieurs parties flexibles (248), ou
- une longueur axiale totale de la partie proximale (242) est non flexible et droite.

5. Ensemble formant tige de pose (200) selon l'une quelconque des revendications 1 à 4, dans lequel chaque partie flexible (248) comprend une ou plusieurs fentes de flexibilité (252)

6. Ensemble formant tige de pose (200) selon l'une quelconque des revendications 1 à 5, dans lequel l'ouverture d'observation (258) est un orifice axialement longitudinal (256-a) disposé sur un côté antérieur (52) du tube (250).

7. Ensemble formant tige de pose (200) selon l'une quelconque des revendications 1 à 6, dans lequel :
- les fentes de flexibilité (252) dans la section distale flexible (244) sont agencées en une rangée (262) circonscrite dans une moitié postérieure (54) de la section transversale du tube (250)
- la paroi (224) du tube (250) dans la section distale flexible (244) est pourvue, en outre, d'une pluralité d'orifices de flexibilité (256-b), dits orifices de stent (257, 256-b), qui confèrent, en outre, un aspect partiellement semblable à un stent au tube (250) ainsi que la déformabilité ;
- la pluralité d'orifices de stent (257, 256-b) est disposée en deux rangées axiales (260', 260"), chaque rangée étant placée sur un côté respectif de l'ouverture d'observation (258) et entre la rangée (262) de fentes de flexibilité (252).

8. Ensemble formant tige de pose (200) selon la revendication 7, dans lequel :
- la plus grande partie ou la totalité des orifices de stent (257, 256-b) présentent la même forme, et/ou
- la plus grande partie ou la totalité des orifices de stent (257, 256-b) présentent la même taille, et/ou
- la plus grande partie ou la totalité des orifices de stent (257, 256-b) présentent la forme d'un triangle, d'un losange, d'un pentagone, d'un hexagone ou d'un polygone.

9. Ensemble formant tige de pose (200) selon l'une quelconque des revendications 1 à 8, dans lequel :
- le tube (250), dans la section de pointe distale (246), comprend une paire de fentes ouvertes axialement longitudinales (270-a, 270-b) s'étendantjusqu'à l'extrémité terminale distale (251) du tube (250) qui définissent une paire de mâchoires de restriction (272, 274) ;
- une mâchoire est une mâchoire (272) en position antérieure (52) et l'autre mâchoire est une mâchoire (274) en position postérieure (54) ;
- la mâchoire antérieure (272) et/ou la mâchoire postérieure (274) sont inclinées vers un état fermé en direction d'un axe central (a-a') du tube (250) ; et
- la mâchoire antérieure (272) et/ou la mâchoire postérieure (274) sont pourvues d'une pluralité d'entailles radiales qui forment une charnière souple (276).

10. Ensemble formant tige de pose (200) selon la revendication 9, dans lequel une extrémité fermée d'une fente ouverte axialement longitudinale (270-a, 270-b) se trouve dans la section de pointe distale (246).

11. Ensemble formant tige de pose (200) selon l'une quelconque des revendications 1 à 5, dans lequel :
- le tube (250) comprend une paire de fentes ouvertes axialement longitudinales (280-a, 280-b) disposées sur un côté antérieur (52) et un côté postérieur (54) du tube (250) et s'étendant jusqu'à l'extrémité terminale distale (251) du tube (250) qui définissent une paire de bras de retenue (282, 284) conçus pour retenir l'implant (230) avant sa mise en place ;
- l'ouverture d'observation (258) est l'une des fentes ouvertes axialement longitudinales (280-a) disposée sur un côté antérieur (52) du tube (250).

12. Ensemble formant tige de pose (200) selon la revendication 11, dans lequel une extrémité fermée (253) d'une fente ouverte axialement longitudinale (280-a, 280-b) se trouve dans la section proximale (242) ou dans la section distale flexible (244).

13. Ensemble formant tige de pose (200) selon l'une quelconque des revendications 1 à 12, dans lequel le tube (250) est biseauté au niveau de la section de pointe distale (246).

14. Procédé de fabrication de l'ensemble formant tige de pose (200) selon l'une quelconque des revendications 1 à 13 comprenant :
- préparer la tige de pose (220) comportant une extrémité proximale (20) et une extrémité distale (20) comprenant la lumière (222) conçue pour maintenir l'implant (230), la tige de pose (220) étant composée d'un matériau flexible mis sous forme de tube résistant à la déformation (250) ;
- créer une courbure au niveau de l'extrémité distale de la tige de pose (220) de façon à former ainsi une section distale flexible (244) ;
- former une extrémité pointue atraumatique dans la section de pointe distale (246) ;
- créer la pluralité de fentes de flexibilité (252) et/ou d'orifices de flexibilité (256-b) dans une section distale flexible (244) de façon à conférer la flexibilité et la déformabilité dans le 1^{er} plan (60) par un enlèvement de matière du tube au niveau de la paroi du tube (224) ;
- créer l'ouverture d'observation (258) par un enlèvement de matière du tube au niveau de la paroi du tube (224).

15. Procédé selon la revendication 14, dans lequel les fentes ouvertes axialement longitudinales (270-a, 270-b, 280-a, 280-b) et/ou la charnière souple (276) sont créées par une étape d'enlèvement de matière du tube au niveau de la paroi du tube (224).
